# EUROPÄISCHE PATENTSCHRIFT

(11) **EP 0 859 764 B1**
(45) Veröffentlichungstag und Bekanntmachung des Hinweises auf die Patenterteilung: **10.11.2004**
(21) Anmeldenummer: 96932551.3
(22) Anmeldetag: 18.09.1996
(51) Int. Cl.: C07D 241/04

(54) **VERFAHREN ZUR KONTINUIERLICHEN HERSTELLUNG VON BASISCHEN, CYCLISCHEN, OPTISCH AKTIVEN alpha-AMINOSÄUREN**
PROCESS FOR THE CONTINUOUS PRODUCTION OF BASIC CYCLIC OPTICALLY ACTIVE alpha-AMINO ACIDS
PROCEDE DE FABRICATION EN CONTINU D'alpha-AMINOACIDES CYCLIQUES BASIQUES OPTIQUEMENT ACTIFS

(30) Priorität: 30.09.1995 DE 19536658
(43) Veröffentlichungstag der Anmeldung: 26.08.1998
(73) Patentinhaber: Degussa AG, 40474 Düsseldorf (DE)
(72) Erfinder: KOTTENHAHN, Matthias, D-63579 Freigericht (DE); STINGL, Klaus, D-63755 Alzenau (DE); DRAUZ, Karlheinz, D-63579 Freigericht (DE)
(86) Internationale Anmeldenummer: PCT/EP1996/004073
(87) Internationale Veröffentlichungsnummer: WO 1997/012881

(56) Entgegenhaltungen:
- WO-A-95/21162
- HELVETICA CHIMICA ACTA, Bd. 43, Nr. 117, 1960, BASEL CH, Seiten 888-896, XP002022196 E. FELDER ET AL.: "Über die katalytische Hydrierung von Pyrazincarbonsäuren" in der Anmeldung erwähnt

## Beschreibung

Die Erfindung betrifft ein Verfahren zur Herstellung von basischen, cyclischen und optisch aktiven α-Aminosäuren der allgemeinen Formel I 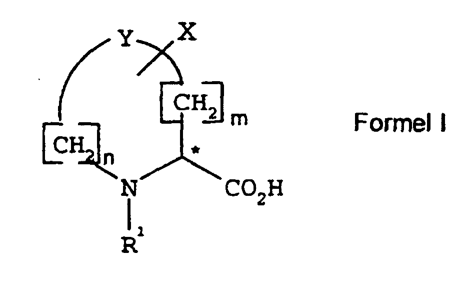 worin
- n, m: unabhängig voneinander 0, 1, 2 oder 3,
- Y: NR¹,
- X: H, NH₂, OH, F, Cl oder Br,
- R¹: H, (C₁-C₆) Alkyl, Benzyl, Formyl, COR² oder CO₂R³,
- R²: (C₁-C₆) Alkyl, Phenyl, Benzyl, NH₂, NO₂-Phenyl oder NO₂-Benzyl,
- R³: (C₁-C₆) Alkyl, Phenyl, Benzyl, NO₂-Phenyl oder NO₂-Benzyl bedeutet und
- *: ein R- oder S-konfiguriertes Kohlenstoffatom kennzeichnet.

Chirale α-Aminosäuren der allgemeinen Formel I sind u. a. wichtige Bausteine für die Pharmaindustrie. So ist z. B. die nichtproteinogene, (S)-konfigurierte Piperazincarbonsäure der Formel II 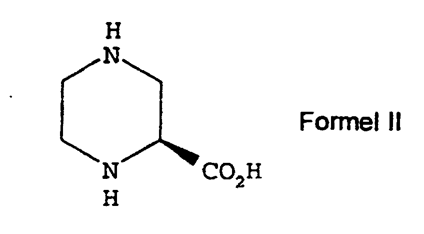 eine Zwischenverbindung des HIV-Proteinase Inhibitors L-735,525 (Tetrahedron Lett. 1994, 35, 673 - 676) der Formel III.

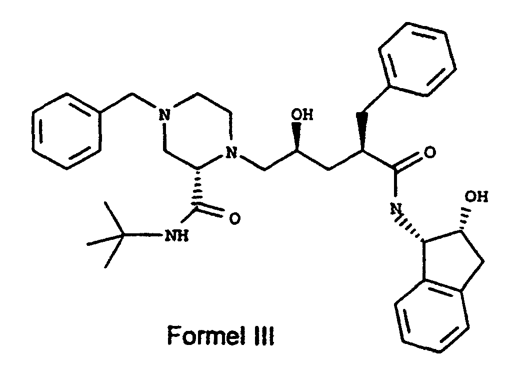

Wie dem Fachmann-allgemein bekannt ist (J. Jacques, Enantiomeres, Racemates and Resolutions, Wiley, New York, 1981), lassen sich racemische Verbindungen der allgemeinen Formel I in verschiedenen Lösungsmitteln mit optisch aktiven Säuren in ihre diastereomeren Salzpaare überführen und durch eine fraktionierende Kristallisation trennen.

Die Synthese der chiralen Aminosäure der Formel II ist beispielsweise in Helv. Chim. Acta 1960, 888 - 896 ausgehend von der aromatischen, heterocyclischen Pyrazincarbonsäure beschrieben. Ein Schlüsselschritt bei der Synthese ist die Racematspaltung der (R,S)-Piperazincarbonsäure über diastereomere Salzpaare mit Hilfe der optisch aktiven (S)-Campfersulfonsäure [(S)-CSA] zum (S,S)-Salzpaar der Formel IV in einer Ausbeute von nur -50 % [bezogen auf ein Diastereomerpaar (eine exakte Ausbeute läßt sich aus der o. g. Literaturstelle nicht entnehmen)], d. h. ca. 25 % Gesamtausbeute.

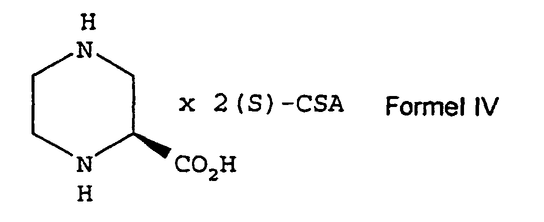

Neben den moderaten Ausbeuten bei einer klassischen Racematspaltung fällt nach Abschluß einer Synthesesequenz i. d. R. eine optische Antipode als Abfallstoff an, so daß diese für technische Prozesse als unwirtschaftlich oder sogar unbrauchbar angesehen werden müssen.

Desweiteren ist bekannt, daß sich chirale α-Aminosäuren durch Zusatz von katalytischen Mengen an Aldehyden (Tetrahedron Lett. 1983, 24, 4457 - 4460), Aldehyden und Metallionen unter alkalischen Bedingungen (JP 42-13445) sowie durch Erhitzen in Wasser unter Druck (US 3 213 106), in starken Basen oder Säuren (A. Neubergerin, M. L. Anson, J. T. Edsall, Advances in Protein Chemistry, Academic Press, New York 1948, 4, S. 339) und in aliphatischen Carbonsäuren (Chem. Pharm. Bull. 1970, 18, 1788 - 1793) racemisieren lassen. Nachteile dieser genannten Racemisierungsmethoden bestehen oftmals in einer zu geringen Racemisierungsrate oder die eingesetzten α-Aminosäuren zersetzen sich partiell unter den Reaktionsbedingungen.

Aufgabe der vorliegenden Erfindung ist es demnach, eine Racematspaltungsmethode für basische, cyclische rac.-Aminosäuren zu finden, die die genannten Nachteile vermeidet und die es ermöglicht, die korrespondierenden optische Antipoden mit Hilfe von chiralen Säuren in Ausbeuten über 50 % zu erhalten, wobei das Hauptaugenmerk auf einer einfachen technischen Durchführbarkeit liegen soll.

Diese Aufgabe wird erfindungsgemäß dadurch gelöst, daß man
a) das Racemat der basischen, cyclischen α-Aminosäure der Formel V 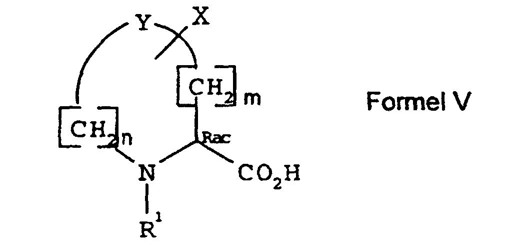 worin n, m, X, Y und R¹ die o. g. Bedeutungen haben und Rac das nahestehende Kohlenstoffatom als racemisch ausweist,
b) mit einer optisch aktiven Hilfssäure zu den Salzpaaren der Formel VI 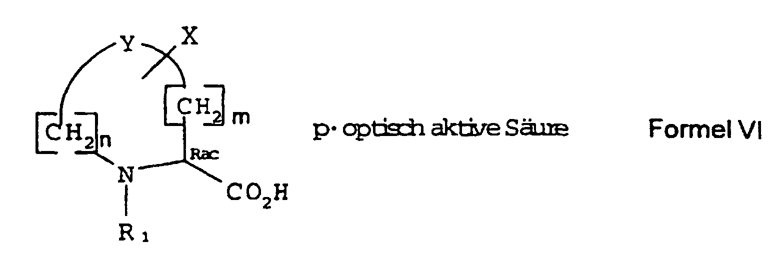 worin n, m, X, Y, R¹ und Rac die o. g. Bedeutungen haben und p das molare Verhältnis der chiralen Säure zur α-Aminosäure angibt, wobei p abhängig von der Anzahl der basischen Zentren ist und eine Zahl von 1 bis 6 sein kann,
   umsetzt, und
c) ein resultierendes diastereomeres Salzpaar der Formel VII 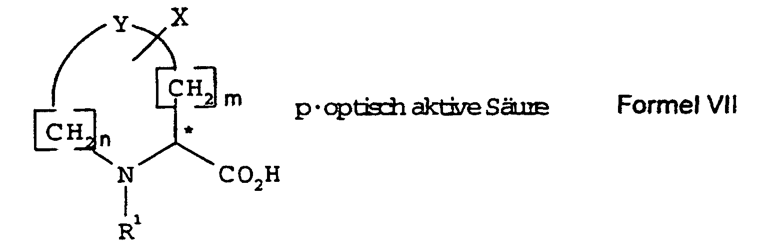 worin n, m, X, Y, R¹, * und p die o. g. Bedeutungen haben,
   von der Mutterlauge trennt und
d) die Mutterlauge mit der entsprechenden zur Racematspaltung eingesetzten chiralen Hilfssäure versetzt und racemisiert, bevor man die Mutterlauge
e) anschließend mit dem Racemat der basischen, cyclischen Aminosäure der Formel V 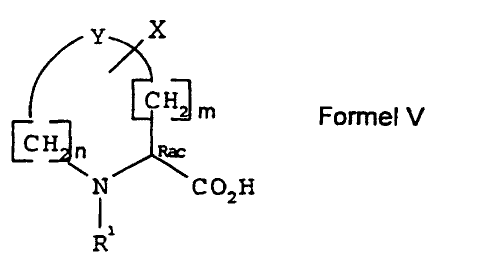 worin n, m, X, Y, Rac und R¹ die o. g. Bedeutungen haben,
   aufstockt und aus den erhaltenen diastereomeren Salzpaaren der Formel VI
f) erneut ein diastereomeres Salzpaar der Formel VII 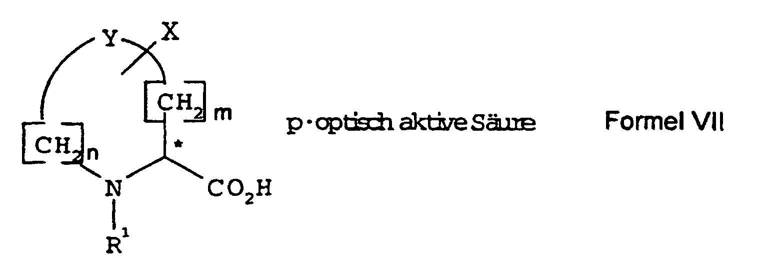 worin n, m, X, Y, R¹, * und p die o. g. Bedeutungen haben,
   von der Mutterlauge abtrennt, mit dem bereits unter c) gewonnenen diastereomeren Salzpaar vereinigt und hiervon
g) die Aminosäure freisetzt.

Überraschenderweise kann erfindungsgemäß die zur Racematspaltung eingesetzte chirale Hilfssäure ebenfalls zur Racemisierung eines diastereomeren Salzpaares verwendet werden, ohne daß ein nennenswerter Verlust an chemischer Ausbeute und chiraler Information eintritt. Dies ist durchaus ungewöhnlich, denn zum einen wird durch die Reaktionsbedingungen und dem Überschuß an chiraler Hilfssäure die α-Aminosäure bzw. das α-Aminosäurederivat zwar racemisiert, die chirale Hilfssäure selbst jedoch nicht.

Vorteilhafterweise kann dabei die Reaktionfolge d) - f) mehrmals hintereinander (kontinuierlich) erfolgen, was ebenfalls ohne einen nennenswerten Verlust an chemischer Ausbeute und chiraler Information erfolgt.

Das vorliegende Verfahren erlaubt eine schonende Reracemisierung der Mutterlaugen durch Zugabe der entsprechenden chiralen Säuren, hohe Ausbeuten an diastereomerem Salzpaar und eine Rückgewinnung der chiralen Hilfsstoffe, wodurch das erfindungsgemäße Verfahren sich als äußerst ökonomisch erweist.

Die anfängliche Trennung der basischen, racemischen Aminosäuren in ihre optische Antipoden nach der klassischen Racematspaltung über ihre korrespondierenden diastereomeren Salzpaare erfolgt nach an sich bekannten Methoden (Bull. Chem. Soc. Jpn. 1989, 62, 109 - 113). Dazu wird das Racemat der Formel I beispielsweise in Wasser, Aceton, Ester, wie beispielsweise Essigsäuremethylester, Essigsäureethylester, Essigsäureisopropylester, oder Alkoholen, wie beispielsweise Methanol, Ethanol, Isopropanol, n-Butanol, tert.Butanol, oder eine Mischung der genannten oder einem anderen polaren Lösungsmittel, vorzugsweise in Wasser gelöst und mit 1 - 10 Äquivalenten, vorzugsweise mit 1 - 6 Äquivalenten einer chiralen Hilfssäure, beispielsweise Enantiomere der Äpfelsäure, Milchsäure, Weinsäure, O,O'-Dibenzoylweinsäure, Ditoluylweinsäure, Pyroglutaminsäure, Bromcamphersulfonsäure, Camphersulfonsäure oder Mandelsäure versetzt. Das kristallisierte diastereomere Salzpaar wird von der Mutterlauge separiert und kann zur Reinigung umkristallisiert werden.

Anschließend wird der Mutterlauge der Racematspaltungslösung die entsprechende gew.-%ige Menge oder ein mehrfaches an chiraler Hilfssäure, die durch Kristallisation des diastereomeren Salzpaares der Mutterlauge entzogen wurde, zugesetzt und der in der Reaktionslösung verbliebene Teil der α-Aminosäure bzw. des α-Aminosäurederivats ggf. bei Temperaturen zwischen 120 °C und 25 °C, bevorzugt zwischen 70 °C und 30 °C, reracemisiert. Bei schwer zu racemisierenden Aminosäuren kann es vorteilhaft sein, der Reaktionslösung 0.01 - 3 Äquivalente, bevorzugt 0.05 - 0.1 Äquivalente, eines Aldehyds, wie beispielsweise Salicylaldehyd oder Benzaldehyd, zuzusetzen.

Danach stockt man die Lösung mit racemischer Aminosäure bzw. einem Aminosäurederivat der Formel I zu den anfänglichen molaren Verhältnissen oder ein mehrfaches wieder auf und impft ggf. mit dem gleichsinnig konfiguriertem Salzpaar an.

Die Freisetzung der entsprechenden chiralen Aminosäure der Formel I aus ihrem diastereomeren Salzpaar der Formel VII erfolgt ebenfalls nach an sich bekannten Methoden (Helvetica Chim. Acta 1960, 888 - 896). Dazu wird das diastereomere Salz der Formel VII beispielsweise in Wasser, Aceton, Ester, wie beispielsweise Essigsäuremethylester, Essigsäureethylester, Essigsäureisopropylester, oder Alkoholen, wie beispielsweise Methanol, Ethanol, Isopropanol, n-Butanol, tert.Butanol, oder eine Mischung der genannten, vorzugsweise jedoch in Wasser gelöst oder suspendiert und mit einer anorganischen Base, wie beispielsweise Natriumhydrogencarbonat, Natriumcarbonat oder Natronlauge oder einer organischen Base, wie beispielsweise Ammoniak, Triethylamin oder N-Methylmorpholin behandelt und die resultierende Aminosäure der Formel I in Lösung weiterverarbeitet oder per Kristallisation isoliert.

Vorzugsweise wird das diastereomere Salzpaar der Formel VII in H₂O gelöst und in an sich bekannter Weise (s. Helv. Chim. Acta 1960, 888) mit Hilfe eines Ionenaustauschers aufgetrennt.

Besonders bevorzugt wird das diastereomere Salzpaar der Formel VII in H₂O gelöst und auf einen basischen Ionenaustauscher, beispielsweise auf Amberlite IR-120®, aufgegeben. Während die Aminosäure auf diesem Ionenaustauscher adsorbiert und anschließend in bekannter Weise freigesetzt wird, kann die chirale Hilfssäure auf diesem Wege direkt zurückgewonnen werden.

So läßt sich auf präparativ einfachem Weg die chirale Hilfssäure nahezu quantitativ recyclieren, was aus Kostengründen und ökologischer Sichtweise sehr bedeutsam für den Gesamtprozess ist.

Die Verbindungen des Strukturtyps I sind literaturbekannt und analog Helvetica Chim. Acta 1960, 888 - 896 herstellbar.

Die beschriebene Methodik soll an den nachfolgenden Beispielen verdeutlicht werden.

### Ausführungsbeispiele

### Beispiel 1:

### (S)-Piperazincarbonsäure x 2(S)-CSA

Zu einer auf 70 °C erwärmten Lösung von 29.3 g (225 mmol) (R,S)-Piperazincarbonsäure und 170 ml H₂O gibt man portionsweise 120.3 g (515 mmol, 2.3 eq.) (S)-Camphersulfonsäure [(S)-CSA] hinzu und läßt auf Raumtemperatur abkühlen. Nachdem man die gelbliche Lösung mit Impfkristallen [(S,S)-Diastereomer] versehen hat, wird sie ca. 16 h zur Kristallisation stehen gelassen. Die sich ausgebildeten harten, farblosen Kristalle werden abgenutscht und mit 20 ml techn. EtOH gewaschen (Wichtig: Die ethanolische Waschlösung nicht mit der Mutterlauge vereinigen). Das Diastereomerenverhältnis des Kristallisats [diastereomeres Salzpaar mit (S,S)-Konfiguration] wird per chiralem HPLC detektiert. Nach Trocknung der Kristalle erhält man 32.7 g Produkt. Die Mutterlauge wird nun mit 25.6 g (S)-CSA aufgestockt und 6 h unter Rückfluß erhitzt. Danach fügt man in der Wärme (70 - 90 °C) 7.15 g rac. (R,S)-Piperazincarbonsäure hinzu und läßt auf Raumtemperatur abkühlen. Es wird erneut angeimpft, zur Kristallisation stehengelassen und wie oben analog aufgearbeitet. Diese Sequenzen werden nun noch weitere viermal durchgeführt, deren Ergebnisse unten aufgeführt sind.

| Sequenz | chem. Ausbeute an (S,S)-Diast. [%] | dv-Wert [(S,S) : (R,S)]* |
|---|---|---|
| 1 | 46 | 99.7 : 0.3 |
| 2 | 42 | 99.5 : 0.5 |
| 2 | 40 | 99.5 : 0.5 |
| 4 | 49 | 99.7 : 0.3 |
| 5 | 42 | 99.7 : 0.3 |
| 6 | 42 | 99.3 : 0.7 |

| | | |
|---|---|---|
| * per chiralem HPLC bestimmt | | |

Reinheit: >95 % per ¹H-NMR

### Beispiel 2:

### (S)-Piperazincarbonsäure

71.4 g (120 mmol) (S)-Piperazincarbonsäure x2(S)-CSA werden in 300 ml H₂O gelöst und über den Kationenaustauscher Amberlite IR 120® (480 ml) geleitet. Anschließend wäscht man den Ionenaustauscher neutral, dampft die Eluate bis zur Trockene ein und erhält nach Vakuumtrocknung (S)-Camphersulfonsäure zu 54.1 g (97 %) enantiomerenrein recycliert. Der Ionenaustauscher wird anschließend nacheinander mit 1.2 1 5 %iger NH₃-Lösung und H₂O eluiert. Die vereinigten Eluate werden bis zur Trockene eingeengt. Der resultierende farblose Feststoff wird in ca. 50 ml warmen H₂O gelöst und das Produkt mit techn. Ethanol unter Rühren gefällt. Die freie enantiomerenreine Aminosäure wird abgenutscht und im Vakuumtrockenschrank bei 60 °C getrocknet.
Ausbeute: 15.14 g (97 %)
Reinheit: >95 % nach ¹H-NMR.
Enantiomerenreinheit: >99 %.

## Patentansprüche

1. Verfahren zur Herstellung von basischen, cyclischen, optisch aktiven, α-Aminosäuren der allgemeinen Formel I 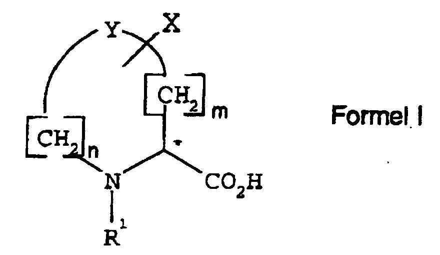 worin
n, m unabhängig voneinander 0, 1, 2 oder 3,
Y NR¹ , wobei
X H, NH₂, OH, F, Cl oder Br,
R¹ H, (C₁-C₆) Alkyl, Benzyl, Formyl, COR² oder CO₂R³,
R² (C₁-C₆) Alkyl, Phenyl, Benzyl, NH₂, NO₂-Phenyl oder NO₂-Benzyl,
R³ (C₁-C₆) Alkyl, Phenyl, Benzyl, NO₂-Phenyl oder NO₂-Benzyl bedeutet und
* ein R- oder S-konfiguriertes Kohlenstoffatom kennzeichnet,
**dadurch gekennzeichnet,**
**daß** man
a) das Racemat der basischen, cyclischen α-Aminosäure der Formel V 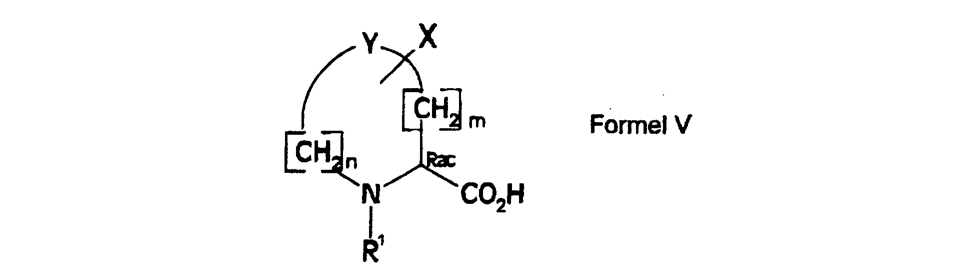 worin n, m, X, Y und R¹ die o. g. Bedeutungen haben und Rac das nahestehende Kohlenstoffatom als racemisch ausweist,
b) mit einer optisch aktiven Hilfssäure zu den Salzpaaren der Formel VI 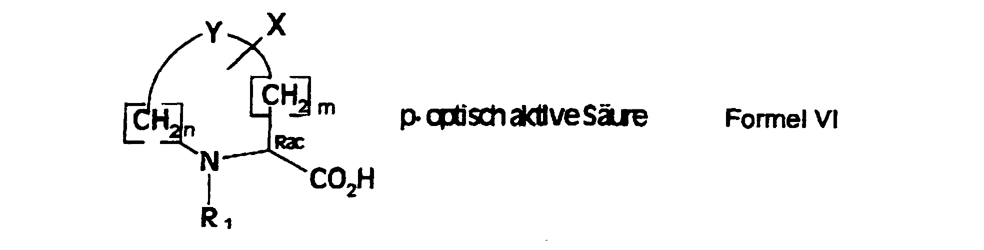 worin n, m, X, Y, R¹ und Rac die o. g. Bedeutungen haben und p das molare Verhältnis der chiralen Säure zur α-Aminosäure angibt, wobei p abhängig von der Anzahl der basischen Zentren ist und eine zahl von 1 bis 6 sein kann,
umsetzt, und
c) ein resultierendes diastereomeres Salzpaar der Formel VII 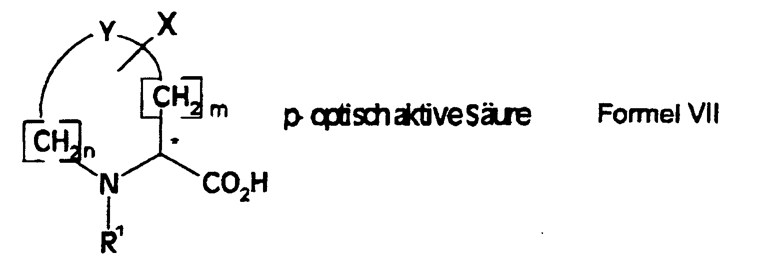 worin n, m, X, Y, R¹, * und p die o. g. Bedeutungen haben,
von der Mutterlauge trennt und
d) die Mutterlauge mit der entsprechenden zur Racematspaltung eingesetzten chiralen Hilfssäure versetzt und racemisiert, bevor man die Mutterlauge
e) anschließend mit dem Racemat der basischen, cyclischen Aminosäure der Formel V 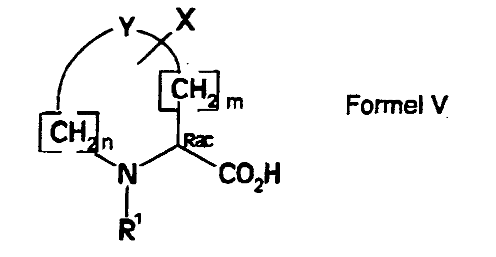 worin n, m, X, Y, Rac und R¹ die o. g. Bedeutungen haben,
aufstockt und aus den erhaltenen diastereomeren Salzpaaren der Formel VI
f) erneut ein diastereomeres Salzpaar der Formel VII 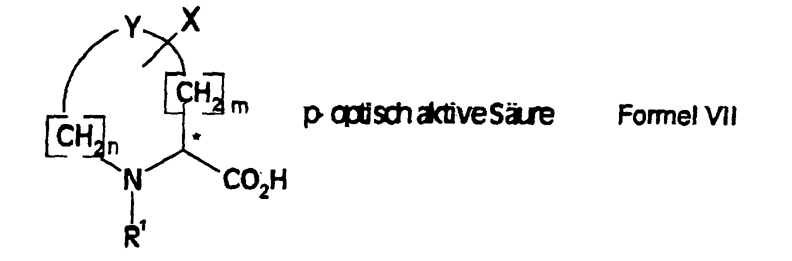 worin n, m, X, Y, R¹, * und p die o. g. Bedeutungen haben,
von der Mutterlauge abtrennt, mit dem bereits unter c) gewonnenen diastereomeren Salzpaar vereinigt und hiervon
g) die Aminosäure freisetzt.

2. Verfahren nach Anspruch 1,
**dadurch gekennzeichnet,**
**daß** als α-Aminosäure (R,S) Piperazincarbonsäure und als chirale Hilfssäure (S)-Camphersulfonsäure verwendet wird.

3. Verfahren nach Anspruch 1,
**dadurch gekennzeichnet,**
**daß** die Reaktionfolge d) - f) mehrmals hintereinander (kontinuierlich) erfolgen kann.

4. Verfahren nach Anspruch 1,
**dadurch gekennzeichnet,**
**daß** man als Lösungsmittel für die fraktionierende Kristallisation der diastereomeren Salzpaare Wasser, Aceton, Ester oder Alkohole oder eine Mischung der genannten einsetzt.

5. Verfahren nach Anspruch 1,
**dadurch gekennzeichnet,**
**daß** man zur Racematspaltung als optisch aktive Säure Enantiomere der Äpfelsäure, Milchsäure, Weinsäure, O,O'-Dibenzoylweinsäure, Ditoluoylweinsäure, Pyroglutaminsäure, Bromcamphersulfonsäure, Camphersulfonsäure oder Mandelsäure verwendet.

6. verfahren nach Anspruch 1,
**dadurch gekennzeichnet,**
**daß** zur Racemisierung der Aminosäure bzw. Aminosäurederivates des in der Mutterlauge verbliebenen Salzpaares die Menge an optisch aktiver Säure zugesetzt wird, die der Reaktionslösung durch Kristallisation des diastereomeren Salzpaares entzogen wurde.

7. Verfahren nach Anspruch 1,
**dadurch gekennzeichnet,**
**daß** die Racemisierung der Aminosäure bzw. Aminosäurederivates des in der Mutterlauge verbliebenen Salzpaares bei Temperaturen zwischen 120 °C und 25 °C, erfolgt.

8. Verfahren nach Anspruch 1,
**dadurch gekennzeichnet,**
**daß** man bei der Racemisierung der α-Aminosäuren bzw. α-Aminosäurenderivate der Mutterlaugenlösung einen Aldehyd zusetzt.

9. Verfahren nach Anspruch 8,
**dadurch gekennzeichnet,**
**daß** der Aldehyd in 0.01 - 3 Äquivalenten zugesetzt wird.

## Claims

1. Process for the production of basic, cyclic and optically active α-amino acids of the general formula I 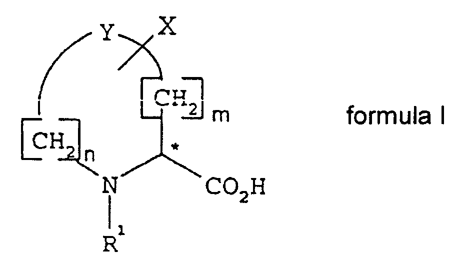 in which
n, m mutually independently mean 0, 1, 2 or 3,
Y NR¹, wherein
X means H, NH₂, OH, F, Cl or Br,
R¹ means H, (C₁-C₆) alkyl, benzyl, formyl, COR² or CO₂R³,
R² means (C₁-C₆) alkyl, phenyl, benzyl, NH₂, NO₂₋ phenyl or NO₂-benzyl,
R³ means (C₁-C₆) alkyl, phenyl, benzyl, NO₂-phenyl or NO₂-benzyl and
* denotes a carbon atom in R or S configuration,
**characterised in that**
a) the racemate of the basic, cyclic α-amino acid of the formula V 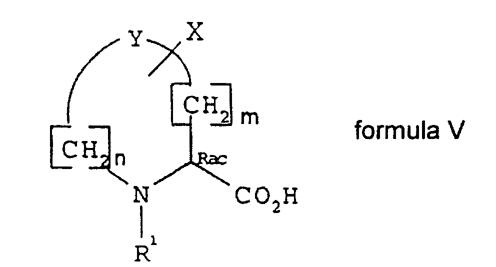 in which n, m, X, Y and R¹ have the above-stated meanings and Rac indicates that the adjacent carbon atom is racemic,
b) is reacted with an optically active auxiliary acid to yield the salt pairs of the formula VI 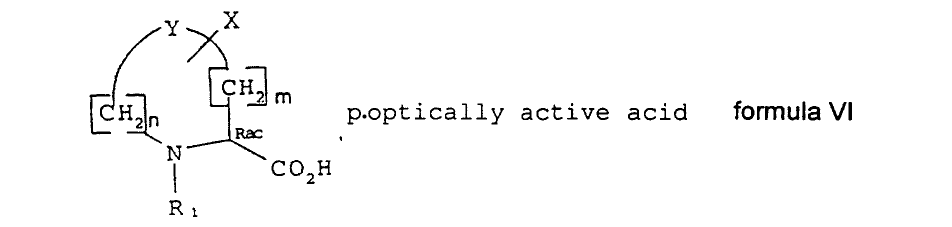 in which n, m, X, Y, R¹ and Rac have the above-stated meanings and p states the molar ratio of the chiral acid to the α-amino acid, wherein p is dependent upon the number of basic centres and may be a number from 1 to 6,
and
c) a resultant diastereomeric salt pair of the formula VII 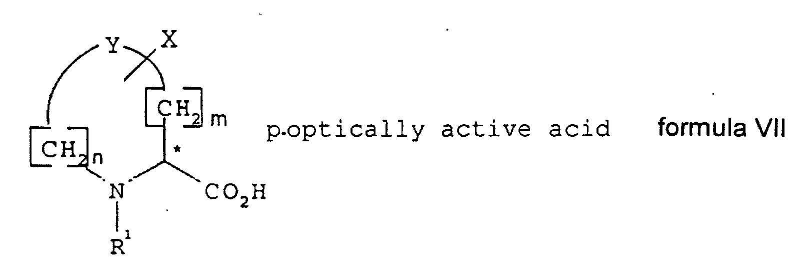 in which n, m, X, Y, R¹, * and p have the above-stated meanings,
is separated from the mother liquor and
d) the mother liquor is combined and racemised with the corresponding chiral auxiliary acid used for resolving the racemate before the mother liquor
e) is subsequently made up with the racemate of the basic, cyclic amino acid of the formula V 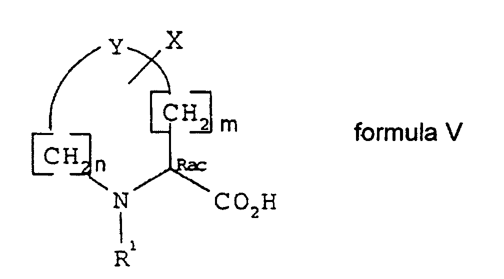 in which n, m, X, Y, Rac and R¹ have the above-stated meanings
and, from the resultant diastereomeric salts pairs of the formula VI,
f) a diastereomeric salt pair of the formula VII is again separated from the mother liquor 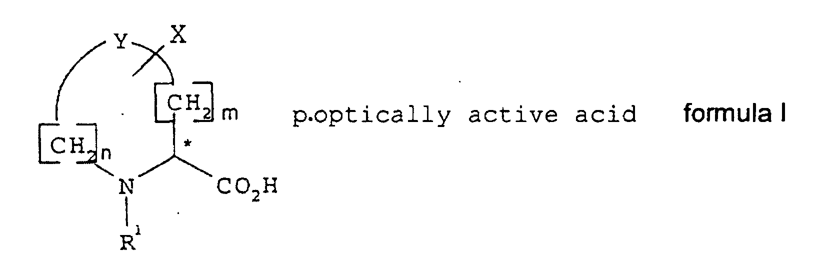 in which n, m, X, Y, R¹, * and p have the above-stated meanings,
this salt pair is combined with the diastereomeric salt pair already obtained in c) and
g) the amino acid is liberated herefrom.

2. Process according to claim 1,
**characterised in that**
(R,S)-piperazinecarboxylic acid is used as the α-amino acid and (S)-camphorsulphonic acid as the chiral auxiliary acid.

3. Process according to claim 1,
**characterised in that**
the reaction sequence d) - f) may proceed repeatedly in succession (continuously).

4. Process according to claim 1,
**characterised in that**
the solvent used for the fractional crystallisation of the diastereomeric salt pairs is water, acetone, esters, isopropyl acetate or alcohols, or a mixture of the stated solvents.

5. Process according to claim 1,
**characterised in that**,
enantiomers of malic acid, lactic acid, tartaric acid, O,O'-dibenzoyltartaric acid, ditoluoyltartaric acid, pyroglutamic acid, bromocamphorsulphonic acid, camphorsulphonic acid or mandelic acid are used as the optically active acid for resolving the racemate.

6. Process according to claim 1,
**characterised in that**
the amino acid or amino acid derivative of the salt pair remaining in the mother liquor is racemised by adding the quantity of optically active acid which was removed from the reaction solution by crystallisation of the diastereomeric salt pair.

7. Process according to claim 1,
**characterised in that**
racemisation of the amino acid or amino acid derivative of the salt pair remaining in the mother liquor proceeds at temperatures of between 120°C and 25°C.

8. Process according to claim 1,
**characterised in that**
an aldehyde is added to the mother liquor solution during racemisation of the α-amino acids or α-amino acid derivatives.

9. Process according to claim 8,
**characterised in that**
0.01 - 3 equivalents of aldehyde are added.

## Revendications

1. Procédé de fabrication d'acides aminés α basiques, cycliques, optiquement actifs, de la formule générale I 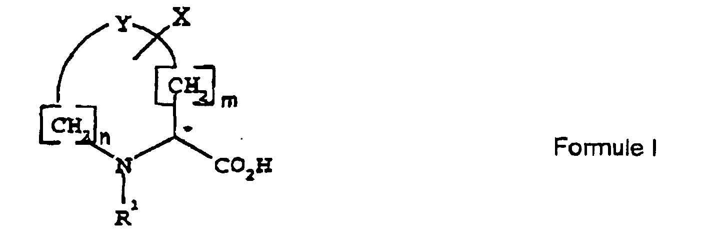 dans laquelle
n, m séparément 0, 1, 2 ou 3,
Y NR¹ dans laquelle
X H, NH2, OH, F, Cl ou Br,
R¹ H, (C₁-C₆) alkyle, benzyle, formyle, COR² ou CO₂R³,
R² (C₁-C₆) alkyle, phényle, benzyle, NH₂, NO₂-phényle ou NO₂-benzyle
R³ (C₁-C₆) alkyle, phényle, benzyle, NO₂-phényle ou NO₂-benzyle et
* représente un atome de carbone en configuration R ou S
**caractérisé par le fait qu'**on fait réagir
a) le racémique de l'acide aminé a basique, cyclique de la formule V 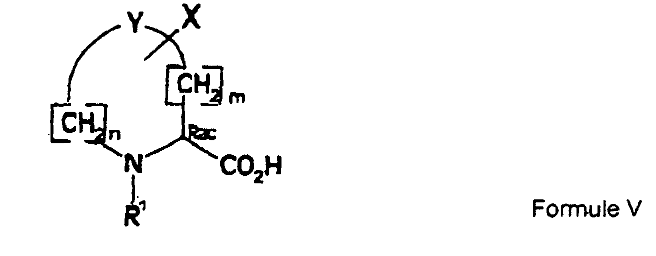 dans laquelle n, m, X. Y et R¹ ont les même significations que ci-dessus et Rac désigne l'atome de carbone proche en tant que racémique,
b) avec un agent résolvant acide optiquement actif dont les entités moléculaires sont de la formule VI 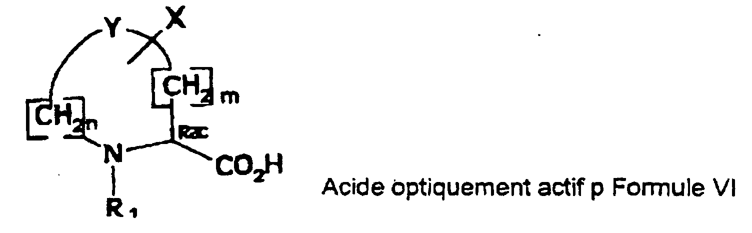 dans laquelle n, m, X, Y , R¹ et Rac ont les mêmes significations que ci-dessus et p indique la proportion moléculaire de l'acide chiral dans l'acide aminé α, auquel cas p dépend du nombre de centres basiques et peut être un nombre compris entre 1 et 6, et
b) il en résulte deux composés diastéréomères de la formule VII 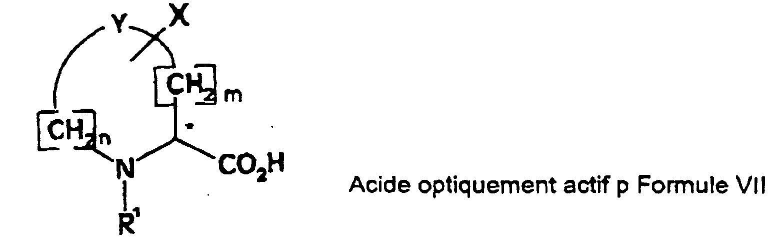 dans laquelle n, m, X, Y, R¹, * et p ont les même significations que ci-dessus,
séparé de la lessive-mère et
d) on mélange et on racémise la lessive-mère avec l'agent résolvant acide chiral correspondant obtenu par dédoublement du racémique avant que la lessive-mère
e) soit ensuite mélangée avec le racémique de l'acide aminé basique cyclique de la formule V 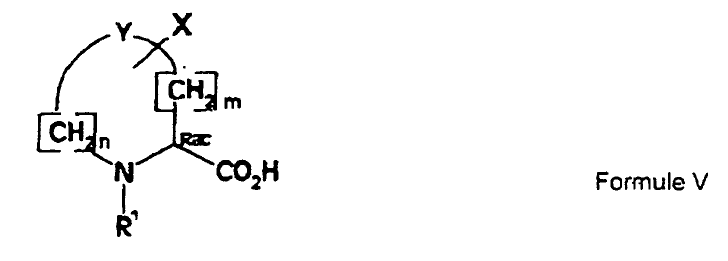 dans laquelle n, m, X, Y, Rac et R¹ ont les même significations que ci-dessus, et à partir des deux composés diastéréomères obtenus de la formule VI
f) on obtient deux composés diastéréomères de la formule VII 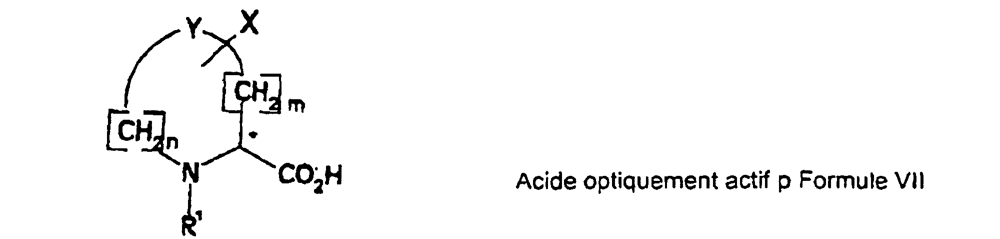 dans laquelle n, m, X, Y, R¹, * et p ont les même significations que ci-dessus, séparés de la lessive-mère, recombinés avec les deux composés diastéréomères c) déjà obtenus et
g) purifiés de l'acide aminé.

2. Procédé selon revendication 1, **caractérisé par** l'utilisation de : l'acide organique de Pipérazine (R,S) en tant qu'acide aminé α et de l'acide sulfonique de Camphre (S) en tant qu'acide chiral.

3. Procédé selon revendication 1, **caractérisé par le fait que** la chaîne de réactions d) - f) peut se produire plusieurs fois de suite (en continu).

4. Procédé selon revendication 1, **caractérisé par** : l'utilisation, comme solvant pour la cristallisation fractionnée des deux composés diastéréomères, d'eau, d'acétone, d'ester, d'alcool ou d'un mélange des éléments cités.

5. Procédé selon revendication 1, **caractérisé par le fait que** : pour le dédoublement du racémique, on utilise en tant qu'acides optiquement actifs, les énantiomères des acides suivants : acide malique, acide lactique, acide tartrique, acide tartrique O,O'-Dibenzoyle, acide tartrique Ditoloyl, acide Pyrroglutamique, acide sulfonique de Camphre bromé, acide sulfonique de Camphre ou acide phénylglycolique.

6. Procédé selon revendication 1, **caractérisé par le fait que** : pour la racémisation de l'acide aminé et/ou du dérivé de l'acide aminé résiduel contenu dans la lessive-mère, la quantité d'acide optiquement actif extraite de la solution réactive par cristallisation des deux composés diastéréomères, est ajoutée.

7. Procédé selon revendication 1, **caractérisé par le fait que** la racémisation de l'acide aminé et/ou du dérivé de l'acide aminé résiduel contenu dans la lessive-mère a lieu à des températures comprises entre 120° C et 25° C.

8. Procédé selon revendication 1 ,**caractérisé par** : l'ajout d'un aldéhyde lors de la racémisation des acides aminés α et/ou des dérivés des acides aminés α à la solution de lessive-mère.

9. Procédé selon revendication 1, **caractérisé par le fait que** l'aldéhyde est rajouté dans la proportion de 0.01 à 3 équivalents.
